# EUROPEAN PATENT APPLICATION

(11) **EP 4 356 884 A1**
(43) Date of publication of application: **24.04.2024**
(21) Application number: 22201831.9
(22) Date of filing: 17.10.2022
(51) Int. Cl.: A61F 13/00, A61F 13/02

(54) **A DRESSING COMPRISING A PATTERNED RELEASE LINER**

(71) Applicant: Mölnlycke Health Care AB, 402 52 Göteborg (SE)
(72) Inventor: JOHANNISON, Ulf, 438 91 LANDVETTER (SE); HANSSON, Dennis, 424 56 GUNNILSE (SE); FLACH, Niclas, 441 35 ALINGSÅS (SE)
(74) Representative: Kransell & Wennborg KB

(57) **Abstract**

The present disclosure generally relates to a medical dressing (100) comprising a top layer (101), an adhesive skin contact layer (102) and a release liner (103) detachably attached to at least a portion of the adhesive skin contact layer (102); the release liner (103) having a first side (104) facing the adhesive skin contact layer (102) and an opposing second side (105). The first side (104) of the release liner (103) comprises a plurality of protrusions (106) spaced apart from one another by non-protruding areas (107), wherein the protrusions (106) are detachably attached to the adhesive skin contact layer (102) and wherein the non-protruding areas (107) are unattached to the adhesive skin contact layer (102). The present disclosure also relates to a process for manufacturing a medical dressing (100).

## Description

### TECHNICAL FIELD

The present disclosure generally relates to a medical dressing comprising a top layer, an adhesive skin contact layer and a release liner detachably attached to at least a portion the adhesive skin contact layer. The release liner has a first side facing the adhesive skin contact layer and an opposing second side. The first side of the release liner comprises a plurality of protrusions spaced apart from one another by non-protruding areas. The present disclosure also relates to a process for manufacturing a medical dressing comprising a patterned release liner.

### BACKGROUND

Adhesive medical dressings are frequently used in wound care, both for the purpose of treating wounds and scars and for the purpose of preventing wounds from occurring in the first place.

Various types of dressings exist on the market. Some dressings, commonly referred to as "film dressings", typically comprise a backing layer (top layer) and an adhesive skin contact layer. Furthermore, so called "bordered dressings" are also frequently used, and these dressings comprise an absorbent pad arranged between the backing layer and the adhesive skin contact layer. In a bordered dressing, the backing layer and the adhesive skin contact layer may be configured to extend beyond the contour of the absorbent pad.

The purpose of the adhesive skin contact layer is to adhere to the skin or the wound of a wearer and to fixate the dressing in a desirable position. A desired property of the adhesive skin contact layer when used in a dressing, regardless of being of the film or border type of dressing, is that it should be thin and pliable in order to conform to the contour of the skin.

While thinness and adhesiveness of film dressings and (at least) the border portion of a bordered dressing is desirable, these attributes typically render the dressings difficult to handle and to apply to a patient. The dressings are not self-supporting, and may wrinkle and stick to themselves during application. The wrinkles formed may produce thin channels through which fluid is able to leak. Accordingly, the dressings may become useless, and must ultimately be discarded.

The adhesive surface of a medical dressing is typically covered and protected by a release liner, which is configured to be removed prior to use. However, once the release liner has been partially or fully removed, the adhesive layer can still wrinkle and adhere to itself. Furthermore, the edges of the adhesive skin contact layer (and the dressing) tend to curl.

Adhesive medical dressings are often applied following surgery, and should initially be sterile for medical applications. Sterile application of the dressing is key to avoid the risk of wound infections. Users, such as clinicians must often apply the dressings under strict aseptic conditions, particularly if the dressings are used in a surgical setting. It is important not to touch the surface of the dressing in order to avoid bacterial contamination.

Various attempts have been made to facilitate the handling and application of thin adhesive dressings. For example, US2009/0105670 discloses a composite structure comprising a cover, a stiffener and a releasable liner. The stiffener and the releasable liner, which comprises three laterally located sections, provide stiffness to the composite structure and prevents the dressing from sticking to itself and wrinkle during application. The stiffener is releasably secured to the upper surface of the cover.

EP3373874B1 discloses a bordered dressing comprising a release liner releasably attached to an adhesive skin contact layer. The release liner comprises at least two removable portions separated by a dividing line provided at a distance of less than 15 mm from the wound pad edge. The provision of the dividing line close to the wound pad edge allows the dressing to be arranged at the end of an incision, and facilitates the application procedure of the dressing onto the skin of a patient.

While the above described documents offer solutions to the problem of handling thin film dressings or bordered dressings with thin border portions, there are still improvements to be made in this field.

Particularly, there is a need to improve and to simplify the procedure to handle and apply thin adhesive dressings for a caregiver in order to avoid, or at least significantly reduce, the formation of wrinkles while also preventing bacterial contamination during application onto the skin of a patient.

### SUMMARY

In view of the above-mentioned problems, it is an object of the present disclosure to provide improvements in the field of adhesive medical dressings and to provide a wrinkle-free and sterile handling and application of such dressings for a caregiver.

According to a first aspect of the present disclosure, there is provided a medical dressing comprising a top layer, an adhesive skin contact layer and a release liner detachably attached to at least a portion of the adhesive skin contact layer; the release liner having a first side facing the adhesive skin contact layer and an opposing second side, wherein the first side of the release liner comprises a plurality of protrusions spaced apart from one another by non-protruding areas, wherein the protrusions are detachably attached to the adhesive skin contact layer and wherein the non-protruding areas are unattached to the adhesive skin contact layer.

The present disclosure is based on the realization that a patterned release liner as described hereinabove significantly improves and facilitates the handling of the dressing prior to and during application of the dressing onto the skin of a patient. More particularly, the dressing of the present disclosure significantly reduces the formation of wrinkles when the release liner is detached from the adhesive skin contact layer and when the dressing is applied to the skin of a patient.

The protrusions form the contact surface with the adhesive skin contact layer, but the non-protruding areas remain unattached to the adhesive skin contact layer. With a conventional dressing, the release liner is typically flat and fully adherent to the adhesive dressing surface. When such a release liner is to be removed from the adhesive surface, a large removal force is required, which almost immediately results in the formation of wrinkles.

As mentioned hereinbefore, adhesive medical dressings are typically thin and flimsy, and very difficult to handle since they are extremely prone to wrinkle formation. In contrast to conventional plasters or band-aids, where the release liner is fully removed prior to application of the dressing, medical dressings used in a care facility and/or a hospital setting, are applied in a different manner. The removal of the release liner is typically performed in a gradual or step-wise manner while simultaneously applying the dressing onto the skin or wound of a patient. The application and handling of the dressing is performed by a caregiver.

The medical dressing of the present disclosure yields a reduced attachment between the release liner and the adhesive skin contact layer. The delamination force; i.e. the force required to remove the release liner, is significantly reduced. The reduced delamination force prevents wrinkles from occurring when peeling off the release liner and applying the dressing onto the skin of a patient. The release liner is still sufficiently adherent and attached to the adhesive skin contact layer in order to prevent the entry of contaminants into the dressing.

The non-protruding areas form gaps between the release liner and the adhesive skin contact layer.

This allows the non-protruding areas to remain unattached to the adhesive skin contact layer during transport and storage. The adhesive skin contact layer does not follow the contour of the patterned release liner and is prevented from "sinking" into the gaps, which is often the case with adhesives utilized in medical dressings. After assembly of the medical dressing, the dressing (including the release liner) is subject to sterilization, which may be regarded as a relatively harsh treatment. During sterilization, the layers of the dressing are pressed together. Such pressing and bringing the layers in closer contact with one another may also occur during transport and storage. However, the provision of gaps between the adhesive skin contact layer and the release liner prevents the release liner from becoming fully attached to the adhesive skin contact layer.

In exemplary embodiments, the first side of the release liner is detachably attached to from 10 to 80 %, preferably from 15 to 70%, most preferably from 20 to 50% of the surface area the adhesive skin contact layer.

Accordingly, the release liner serves the dual function of protecting the adhesive skin contact layer, yet facilitating the removal of the release liner such that wrinkles are prevented from forming during removal of the release liner.

In exemplary embodiments, each of the plurality of protrusions has a substantially flat top surface; the top surface forming the contact surface for detachable attachment of the release liner to the adhesive skin contact layer.

This is beneficial to secure that the adhesive skin contact layer does not "sink" into the "valleys" defined by the non-protruding areas, e.g. during sterilization, transport or storage of the medical dressing. Soft adhesives, such as silicone-based adhesives, have a tendency to follow the contour of an underlying surface. This is a desired property when the dressing is to be applied onto the skin of a patient, but undesired for the purpose of preventing wrinkles during removal of the release liner.

The flat top surface of the protrusions secure that the gaps defined between the adhesive skin contact layer and the release liner are maintained, and that a significant portion of the release liner remains unattached to the adhesive skin contact layer. Instead of sinking into the valleys defined by the non-protruding areas, which would be the result if e.g. a curvy protruding profile was utilized, the adhesive skin contact layer lies flat on top of the plurality of protrusions.

Furthermore, if the top surface of the protrusions is angled or sharp, the protrusions may create indentations and imprints in the adhesive skin contact layer, which is undesirable when the dressing is attached to the skin. Such imprints may reduce the adherence to the skin of a patient, and the ability of the adhesive skin contact layer to conform to the skin may be impaired.

In exemplary embodiments, each of the plurality of protrusions has a top surface and sidewalls extending between the top surface and the non-protruding area, wherein the angle, α, between the top surface and the sidewalls is from 60 to 120 degrees, preferably from 75 to 105 degrees, most preferably from 85 to 95 degrees.

An angle in the above-mentioned range further prevents the adhesive skin contact layer from sinking into the gaps defined between the release liner and the adhesive skin contact layer. Hence, the adhesive skin contact layer remains essentially flat and is prevented from following the contour of the protrusions. This furthermore secures that the adhesive skin contact layer remains unattached to the release liner in the non-protruding areas.

In exemplary embodiments, the height of each of the protrusions may be from 0.3 to 2.0 mm, preferably from 0.5 to 1.5 mm.

These dimensions are beneficial for achieving an appropriate balance of protecting the dressing from becoming contaminated, preventing the formation of imprints in the adhesive skin contact layer, and securing that the non-protruding areas do not become adhesively attached to the adhesive skin contact layer.

In exemplary embodiments, the surface area of the top surface of each of the protrusions may be from 0.8 to 3.0 mm2, preferably from 1.0 to 2.0 mm2.

A surface area in the above-mentioned range improves the detachable attachment to the adhesive skin contact layer. If the surface area is too large, the first side of the release liner may become too firmly attached to the adhesive skin contact layer. As a result, the delamination force may increase (resulting in wrinkles upon removal of the release liner). If the surface area is too small, a soft adhesive skin contact layer may become more prone to sinking into the valleys or gaps defined by the non-protruding areas of the first side of the release liner.

In exemplary embodiments, the adhesive skin contact layer may comprise a silicone-based adhesive coating.

A silicone-based adhesive coating is soft and gentle to the skin and can be removed without causing trauma.

However, the softness of the silicone-based adhesive may render the adhesive skin contact layer more prone to adapt to the contour of an underlying surface structure. As mentioned hereinbefore, this is beneficial in the application against the skin or wound of a patient, particularly a curved or contoured skin surface. However, it is undesired if the adhesive skin contact layer conforms to the tops and valleys defined by the protrusions, and non-protruding areas, respectively, on the first side of the adhesive skin contact layer.

In exemplary embodiments, the adhesive skin contact layer may comprise a polymeric film provided with a silicone-based adhesive coating.

The polymeric film yields stability to the applied silicone-based adhesive coating, and counter-acts the tendency of the silicone adhesive to follow the contour of the protrusions. Accordingly, the polymeric film prevents the silicone-based adhesive from sinking deeper into the valleys defined by the non-protruding parts of the first side of the release liner.

In exemplary embodiments, the silicone-based adhesive coating has a thickness of from 35 to gsm to 300 gsm, preferably from 45 to 250 gsm.

If the thickness of the silicone-based adhesive coating is too low, poor adhesion to the skin may result. In contrast, a thicker silicone-based adhesive coating has an increased tendency to follow the contour of the protrusions. Furthermore, the risk of the protrusions forming imprints in the silicone-based adhesive skin contact layer is enhanced with a thicker silicone-based coating.

In exemplary embodiments, the dressing may further comprise an absorbent pad arranged between the top layer and the adhesive skin contact layer, wherein the top layer and the adhesive skin contact layer are configured to extend beyond the contour of the absorbent pad to define a border portion along the contour of the absorbent pad.

Accordingly, the medical dressing may be a bordered dressing. The absorbent pad serves the purpose of dealing with large amounts of wound liquid exuded by the wound. With a bordered dressing, the border portion is particularly susceptible to wrinkle formation, and easily crinkles and adheres to itself once the release liner is removed. It is therefore beneficial to arrange the release liner such that the plurality of protrusions covers the adhesive skin contact layer in at least the border portion of the dressing. The absorbent pad may yield a certain level of rigidity to the overall dressing structure, and in this regard, facilitate the procedure of removing the release liner and applying the dressing for a caregiver.

The plurality of protrusions on the first side of the release liner may be provided by any technique known in the art.

In exemplary embodiments, the plurality of protrusions may be formed by embossing.

Embossing is a simple and reliable technique enabling the provision of distinct, three dimensional protrusions in the release liner that remain in place during e.g. storage and transport of the dressing.

According to another aspect, there is provided a process for manufacturing a medical dressing comprising:
a) providing a dressing comprising a top layer and an adhesive skin contact layer,
b) providing a release liner having a first side and an opposing second side, wherein at least a portion of the first side of the release liner comprises a plurality of protrusions spaced apart from one another by non-protruding areas;
c) applying the release liner onto the adhesive skin contact layer such that the plurality of protrusions become detachably attached to the adhesive skin contact layer and unattached to the adhesive skin contact layer in the non-protruding areas.

In exemplary embodiments, the protrusions on the first side of the release liner are formed as an in-line step in the process.

This has several advantages from a process and storage perspective. If the protrusions are provided in the release liner as a separate process step prior to the process of manufacturing the dressing, the protruded release liner would require much more space during storage; e.g. when stored on a storage roll. In contrast, if the plurality of protrusions are formed as an in-line step, the release liner may be stored on storage rolls in a flat condition.

In exemplary embodiments, the plurality of protrusions on the first side of the release liner may be formed by embossing.

As mentioned hereinbefore, embossing is a simple technique to form three dimensional features, e.g. protrusions in a layer. Furthermore, embossing is suitable for use in embodiments where the protrusions are formed as an in-line step in the process.

Further features of, and advantages with, the present disclosure will become apparent when studying the appended claims and the following description. The skilled addressee realizes that different features of the present disclosure may be combined to create embodiments other than those described in the following, without departing from the scope of the present disclosure.

### BRIEF DESCRIPTION OF THE DRAWINGS

The various aspects of the present disclosure, including its particular features and advantages, will be readily understood from the following detailed description and the accompanying drawings, in which:
Figure 1a illustrates a medical dressing according to an exemplary embodiment of the present disclosure, as seen from the bottom side when the release liner is to be peeled off by a caregiver.
Figure 1b is a zoomed-in view of a portion of the dressing in figure 1a.
Figure 1c is a cross-sectional view of a portion of the medical dressing in figure 1a, showing the release liner being peeled off from the adhesive skin contact layer.
Figure 2 illustrates a medical dressing according to an exemplary embodiment of the present disclosure.
Figures 3a and 3b illustrate the application procedure of a medical dressing according to the present disclosure, as performed by a caregiver.
Figure 4a illustrates a scanning electron microscope (SEM) image of a release liner having a pyramidal shaped protrusion pattern, used for Dressings B and E in Example 1.
Figure 4b illustrates a SEM image of the adhesive skin contact layer of Dressing B (Example 1) after the release liner has been peeled off.
Figure 4c illustrates a SEM image of a cross-sectional view of Dressing B in Example 1.
Figure 4d illustrates a SEM image of a cross-sectional view of Dressing C; i.e. a dressing according to a preferred embodiment of the present disclosure, in Example 1.

### DETAILED DESCRIPTION

The present disclosure will now be described more fully hereinafter with reference to the accompanying drawings, in which currently preferred embodiments of the present disclosure are shown. The present disclosure may, however, be embodied in many different forms and should not be construed as limited to the embodiments set forth herein; rather, these embodiments are provided for thoroughness and completeness, and fully convey the scope of the present disclosure to the skilled person. Like reference characters refer to like elements throughout.

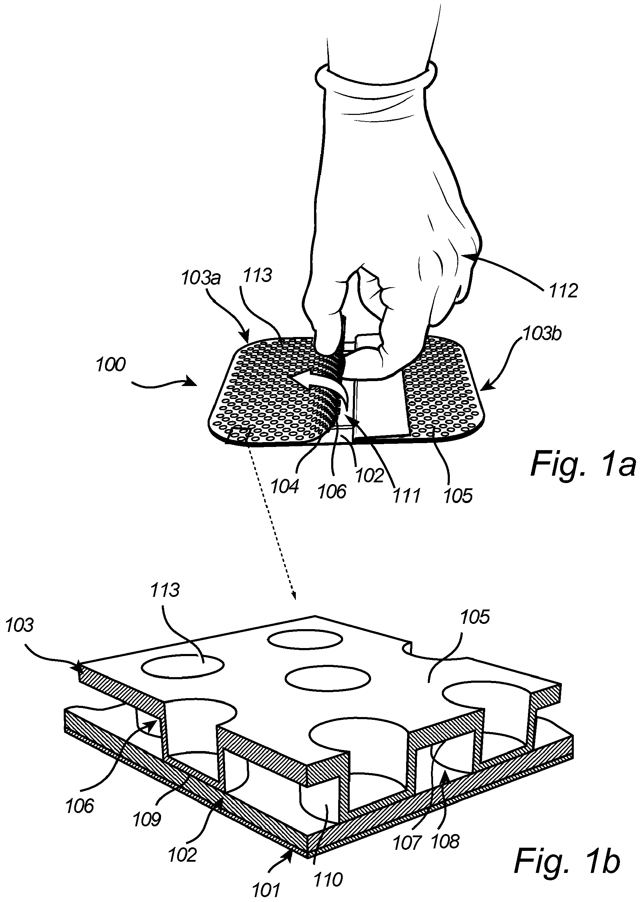

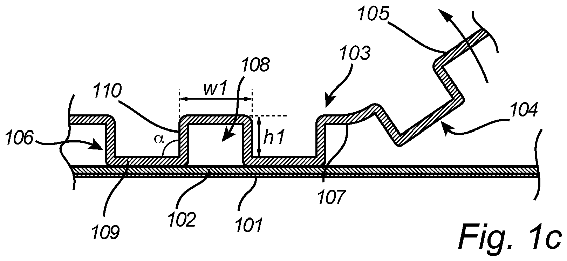

Figure 1a illustrates a medical dressing according to an exemplary embodiment of the present disclosure, wherein the release liner is about to be peeled off by a caregiver. Figure 1b is a zoomed in view of the dressing in figure 1a, and figure 1c illustrates a cross-sectional, partial view of the medical dressing in figure 1a.

The medical dressing 100 comprises a top layer 101, an adhesive skin contact layer 102 and a release liner 103 detachably attached to at least a portion of the adhesive skin contact layer 102. The release liner 103 has a first side 104 facing the adhesive skin contact layer 102 and an opposing second side 105, wherein the first side 104 of the release liner 103 comprises a plurality of protrusions 106 spaced apart from one another by non-protruding areas 107, wherein the protrusions 106 are detachably attached to the adhesive skin contact layer 102 and wherein the non-protruding areas 107 are unattached to the adhesive skin contact layer 102.

As used herein, the term "top layer" means the uppermost layer of the medical dressing; i.e. the layer facing away from the patient's skin during use. The top layer may also be referred to as a backing layer.

The adhesive skin contact layer is the layer that is to be arranged in contact with the patient's skin. The skin contact layer may also be referred to as a wound contact layer.

The top layer and the adhesive skin contact layer are typically co-extensive. The surface area of the top layer is the same as the surface area of the adhesive skin contact layer.

The release liner is arranged to cover the entire surface area of the adhesive skin contact layer.

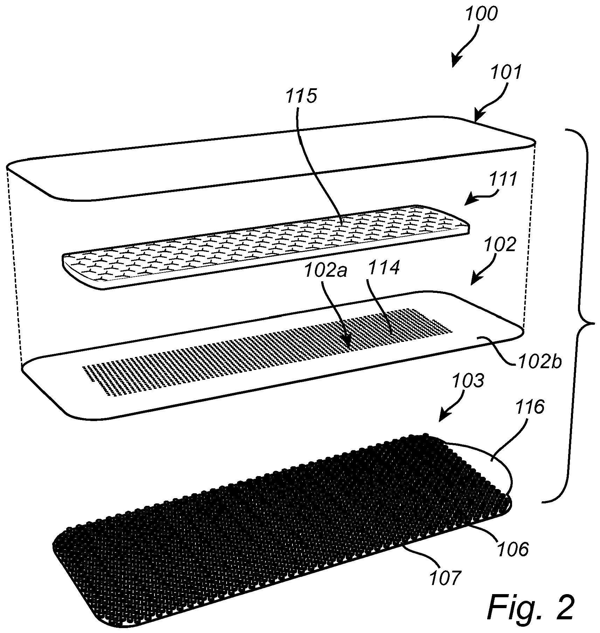

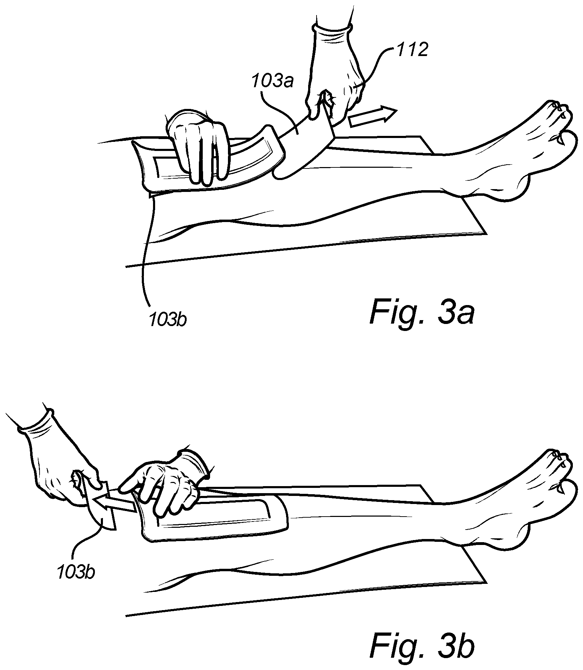

The release liner may comprise one or a plurality of removable portions. In figure 1a (and figures 3a-b), the release liner contains two removable portions (103a and 103b), but the invention is by no means limited to this construction. The dressing illustrated in figure 2 comprises a release liner 103 with one removable portion.

If the first release liner comprises more than one removable portion, the first release liner may be divided by a dividing line, wherein a first 103a and a second 103b removable portion of the release liner overlaps along the dividing line. The overlapping configuration is important to secure that the adhesive skin contact layer is fully covered and that no gaps are formed, thereby preventing contaminants from entering the dressing. The overlapping removable portions may form a grip member that the applicator, e.g. caregiver 112 can grasp in order to remove and separate the removable portion. For example, as illustrated in figure 1a, an edge of the second removable portion 103b may be folded over itself, and the edge of the first removable portion 103a may be configured to overlap the folded edge.

Regardless of how many removable portions the release liner contains, the release liner is arranged to cover the entire adhesive skin contact layer.

The dressing of the present disclosure may be used on all types of wounds, incisions and even on intact skin (if the purpose of the dressing is to prevent wounds from occurring in the first place). The dressing is advantageously used on larger wounds or scars, and may be of a relatively large size.

The application of larger sized dressing is typically more complicated, as a larger sized dressing has an increased tendency to stick to itself, and wrinkles are easily formed, particularly at the edge portion of the dressing. The dressing of the present disclosure is also advantageously used on dressings comprising large and "flimsy" border portions.

It should however be noted that the present disclosure has several advantages with smaller sized dressings as well. For example, a smaller sized border dressing may suffer from the same problem of wrinkle formation and "curling" when the release liner is removed.

Conventional release liners are typically flat; i.e. they are fully adherent to the adhesive skin contact layer prior to use. When such release liners are to be removed from the adhesive skin contact layer prior to and during application to the patient's skin, the force required to remove the release liner is large. Accordingly, the removal of the release liner may give rise to substantial wrinkle formation.

The handling of thin adhesive dressings poses demands on the applicator, which is typically a caregiver (sometimes more than one caregiver). As illustrated in figures 3a and 3b, the removal of the release liner is carried out while simultaneously anchoring the dressing onto the skin of a patient. In figures 3a-3b, the release liner comprises two removable portions (103a and 103b). The first removable portion 103b is first partially removed from the adhesive skin contact layer, and the exposed adhesive surface is positioned onto the wound or incision on the patient (typically at the edge of the incision or wound). While slightly pressing the dressing onto the skin, the caregiver 112 may utilize one hand to gradually remove the first removable portion 103 and simultaneously, with the other hand, anchor the adhesive skin contact layer (and dressing) onto the skin in a smooth and wrinkle-free manner. Since the gradual removal of the release liner requires a significantly reduced force to remove and detach from the adhesive surface, the application of the dressing onto the skin of a patient is greatly facilitated for the caregiver. A more controlled, sterile and wrinkle free application procedure is accomplished.

As best illustrated in figures 1b and 1c, the plurality of non-protruding areas 107 define gaps 108 between the release liner 103 and the adhesive skin contact layer 102.

The distinct gaps between the first side 104 of the release liner 103 and the adhesive skin contact layer 102 secure that the non-protruding areas 107 remain unattached to the adhesive skin contact layer 102.

In figures 1a-1c, the second side 105 of the release liner 103 comprises depressions (denoted 113 in figures 1a and 1b). The depressions 113 on the second side 105 of the release liner 103 coincide with the protrusions 106 on the first side 104 of the release liner 103. The depressions 113 on the second side 105 may be formed when the release liner is embossed. For example, the release liner may be subject to an embossing tool on the second side of the release liner, which form protrusions 106 on the first side 104 and corresponding depressions 113 on the second side 105 of the release liner. The present disclosure is, however, not limited to embossed protrusions. In exemplary embodiments, the second side 105 of the release liner is flat.

The first side 104 of the release liner 103 may be detachably attached to from 10 to 80 %, preferably from 15 to 70%, most preferably from 20 to 50% of the surface area of the adhesive skin contact layer.

Accordingly, the problems associated with a too firm attachment to the adhesive skin contact layer are overcome, and the detachment of the release liner and subsequent application of the dressing onto the skin, is greatly facilitated for a caregiver.

As illustrated in figures 1b and 1c, each of the plurality of protrusions has a substantially flat top surface 109; the top surface 109 forming the contact surface for detachable attachment of the release liner 103 to the adhesive skin contact layer 102.

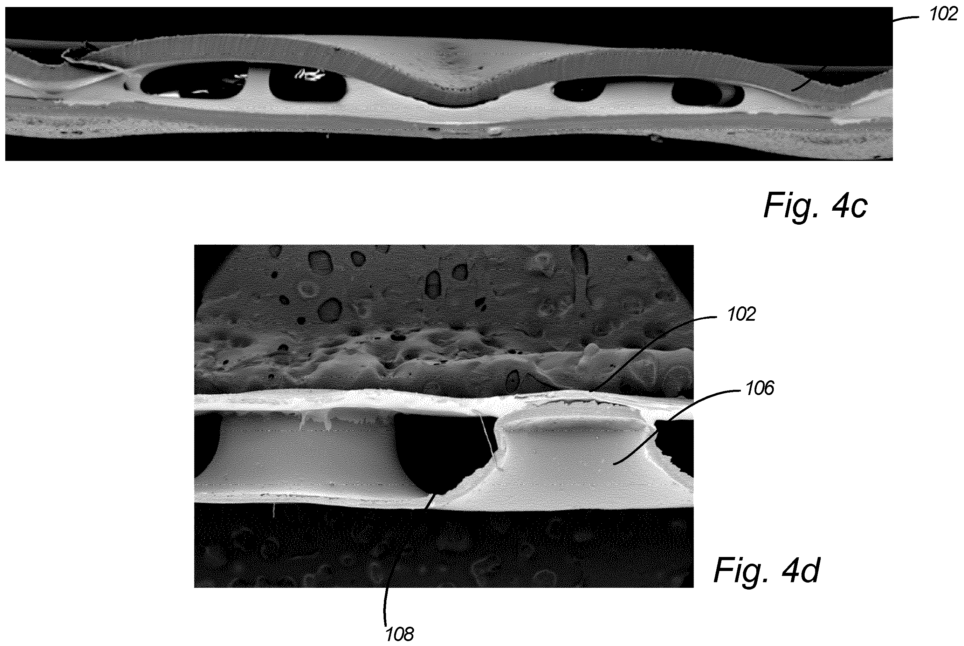

This is considered beneficial for various reasons. First, because imprints in the adhesive skin contact layer caused by too sharp or too angled protrusion top surfaces are avoided. Furthermore, the flat top surface secures that the adhesive skin contact layer lies flat on top of the plurality of protrusions without sinking into the valleys defined by the non-protruding areas. This is evident from the SEM picture in figure 4d, where clear and distinct gaps 108 defined by the non-protruding areas of the first side of the release liner are also illustrated. In contrast, the SEM picture of figure 4c illustrates how the adhesive skin contact layer clearly conforms to the patterned protrusions of the release liner (see Example 1 hereinbelow), and leaves no gaps between the release liner and the adhesive skin contact layer.

As best illustrated in figure 1c, each of the plurality of protrusions 106 has a top surface 109 and and sidewalls 110 extending between the top surface 109 and the non-protruding area 107, wherein the angle, α, between the top surface 109 and the sidewalls 110 is from 60 to 120 degrees, preferably from 75 to 105 degrees, most preferably from 85 to 95 degrees.

If the angle, α, is too large, which may e.g. be the case with a patterned release liner having a "wavy" pattern, the adhesive skin contact layer has a tendency to conform to the contour of the wavy pattern. In this regard, the delamination force between the adhesive skin contact layer and the release liner may actually increase (see Example 1).

The height, h1, of each of the protrusions 106 may be from 0.3 to 2.0 mm, preferably from 0.5 to 1.5 mm.

The height, h1, is the maximum longitudinal extension of the protrusion, and is measured between the top surface 109 of the protrusion and the non-protruding area 107.

These dimensions are suitable for achieving well defined gaps between the protrusions and securing that the adhesive skin contact layer does not conform to or become attached to the release liner in the gaps (non-protruding areas). If the height of the protrusion is too large, the risk of forming imprints in the adhesive skin-contact layer increases. In contrast, if the height is too small, the adhesive skin contact layer may become attached to the entire first side of the release liner, which is undesirable.

The width, w1, of the protrusion may be in the range of from 0.8 to 3.0 mm, e.g. from 1.0 to 2.0 mm.

The width, w1, is the maximum lateral extension of one protrusion 106.

The surface area of the top surface 109 of each protrusion 106 may be from 0.8 to 3.0 mm2, preferably from 1.0 to 2.0 mm2.

A surface area in the above-mentioned range improves the detachable attachment to the adhesive skin contact layer. If the surface area is too large, the first side of the release liner may become too firmly attached to the adhesive skin contact layer. As a result, the delamination force may increase (resulting in wrinkles upon removal of the release liner). If the surface area is too small, the adhesive skin contact layer may become more prone to sinking into the valleys or gaps defined by the non-protruding areas of the first side of the release liner.

The top surface 109 of each protrusion is not limited to a particular shape, but any shape is conceivable, e.g. circular, oval, square, rectangular etc., and. Typically, the top surface is circular.

The plurality of protrusions 106 may be arranged in a pattern extending across the entire surface facing the adhesive skin contact layer 102.

At least 50% of the first side 104 of the release liner 103 may comprise a pattern of protrusions.

If the dressing is a bordered dressing, and comprises a centrally disposed absorbent pad, the plurality of protrusions 106 may be arranged in a pattern extending across the surface facing the adhesive skin contact layer 102 of the border portion.

The plurality of protrusion is typically arranged in a regular pattern across the first side of the release liner.

The distance between one protrusion and an adjacent protrusion may be from 1.0 to 8.0 mm, e.g. from 1.0 to 3.0 mm. The distance between adjacent protrusions depends on the dimensions of the protrusions, the size of the dressing etc. The distance between one protrusion and an adjacent protrusion may correspond to the diameter or the maximal width of the protrusion.

The adhesive skin contact layer 102 may comprise a silicone-based adhesive coating.

Such an adhesive is skin-friendly and permits the removal of the dressing without causing damage to the skin. The softness of the silicone based adhesive may be associated with some challenges since a soft silicone based adhesive has an enhanced tendency to conform to an underlying surface. In the context of the present disclosure, this is avoided by means of the unattached regions of the first side of the release liner that define distinct gaps.

The silicone-based adhesive coating may have a thickness of from 35 to 300 gsm, e.g. from 45 to 250 gsm.

Accordingly, an appropriate balance between skin adhesion, prevention of imprints in the silicone adhesive, and preventing the silicone adhesive from following the contour of the protrusions, is achieved.

The adhesive skin contact layer 102 may comprise a polymeric film provided with a silicone-based adhesive coating (not shown).

The polymeric film yields stability to the applied silicone-based adhesive coating, and prevents the silicone adhesive from following the contour of the protrusions.

The polymeric film is preferably a breathable film and may comprise e.g. polyethylene, polyamide or polyurethane. Preferably, the polymeric film comprises polyurethane. The thickness of the polymeric film may be from 15 to 100 µm, e.g. from 20 to 80 µm, preferably from 20 to 50 µm.

The silicone based adhesive coating of the adhesive skin contact layer is arranged to contact the skin of a wearer.

As best illustrated in figure 2, the medical dressing 100 may further comprise an absorbent pad 111 arranged between the top layer 101 and the adhesive skin contact layer 102, wherein the top layer 101 and the adhesive skin contact layer 102 are configured to extend beyond the contour of the absorbent pad 111 to define a border portion along the contour of the absorbent pad 111.

As illustrated in figure 2, the adhesive skin contact layer may comprise a central portion 102a and an edge portion 102b surrounding the central portion 102a. The surface area of the central portion 102a typically corresponds to the surface area of the absorbent pad 111. The edge portion 102b extends beyond the contour of the absorbent pad 111, and together with the top layer 101, form the border portion of the dressing.

The adhesive skin contact layer 102 may comprise a plurality of perforations 114 in at least the area underlying the absorbent pad 111. In figure 2, the adhesive skin contact layer is void of perforations in the edge portion 102b; i.e. in the area forming the border portion.

The provision of perforations in the adhesive skin contact layer improves the fluid handling within the dressing, and this is equally applicable to the dressings illustrated in figures 1-c.

In the case of a bordered dressing, as illustrated in figure 2, the perforations 114 serve to improve the absorption of wound exudate into the dressing, and are therefore arranged in the area where absorption takes place. The area of the adhesive skin contact layer forming the border portion of the dressing may be void of perforations. This way, the adhesion against the skin is enhanced, and the stay-on ability of the medical dressing is thereby prolonged. However, this construction also renders the border portion flimsier, more prone to wrinkle formation and is more difficult to handle and apply to the skin.

If the adhesive skin contact layer 102 comprises a polymeric film and a silicone-based adhesive coating, the perforations 114 extend through both of these layer.

The absorbent pad 111 may comprise a foam or a gel. It may also comprise a superabsorbent material e.g. superabsorbent polymers (SAP) or superabsorbent fibers (SAF).

For example, the absorbent pad 111 may comprise two or more layers having different properties.

The absorbent pad 111 may comprise a first absorbent layer and a liquid acquisition layer. The absorbent pad may further comprise a second absorbent layer. The first absorbent layer may be a superabsorbent layer and the second absorbent layer may comprise an absorbent foam. Typically, the superabsorbent layer is arranged between the liquid acquisition layer and the second absorbent layer. Suitable foam materials for use in the second absorbent layer include, but are not limited to polyurethane foams.

The superabsorbent layer may comprise a superabsorbent polymer (SAP). A "superabsorbent polymer" or "SAP" is a polymer that can absorb up to 300 times its own weight in aqueous fluids. Superabsorbent polymers are constituted by water-swellable and water insoluble polymers capable of absorbing large quantities of fluid upon formation of a hydrogel. The superabsorbent polymers for use in accordance with the present disclosure may be inorganic or organic crosslinked hydrophilic polymers, such as polyvinyl alcohols, polyethylene oxides, crosslinked polyacrylates and the like. Typically, the superabsorbent (SAP) comprise sodium acrylate. The SAP material may be in the form of particles, fibers, flakes or similar.

The liquid acquisition layer may comprise any material having the ability to distribute the exudate in an efficient manner. For example, the liquid acquisition layer may comprise a nonwoven material. A nonwoven imparts an appropriately balanced rigidity to the layer and to the dressing as such. It may also efficiently distribute and spread liquid absorbed by the superabsorbent layer such that it can be evaporated through the top layer over a large surface. For example, the nonwoven may comprise viscose, polyester or blends thereof.

The layers can be joined by adhesion, lamination, e.g. by using pressure and/or heat.

The absorbent pad 111 may comprise additional layers, such as liquid transport layers, various combinations of foam and nonwoven layers.

As illustrated in figure 2, the absorbent pad 111 may comprise a plurality of cuts 115 extending at least partially through the absorbent pad 111. The cuts 115, which may have various shapes, render the pad more flexible, and enhances the flexibility of the entire dressing.

The cuts 115 illustrated in figure 2 each comprises three incisions extending from a common starting point. The angle between such incisions may be between 40 and 150°. Accordingly, the pad or pad layer(s) is/are cut in both the longitudinal (y) and lateral (x) directions of the pad such that the pad becomes flexible in all directions.

If the absorbent pad 111 comprises several pad-forming layers, the cuts 115 may extend through at least one of such layers.

The absorbent pad 111 may comprise one or more biologically active substance, e.g. a compound having an antimicrobial or wound healing effect. Examples of such compounds include, but are not limited to a silver compound such as silver salt and metallic silver, biguanide salts such as polyhexamethylene biguanide (PHMB) or any salts thereof, or polyhexamethyl guanide (PHMG) or any salts thereof, or chlorhexidine or any salts thereof, iodine, salicylic acid or any salt thereof, acetyl salicylic acid or any salt thereof, quarter ammonium salts such as benzethonium chloride, povidone-iodine (betadine), lactoferrin, xylitol, antimicrobial peptides such as human cationic antimicrobial protein 18 (hCAP18 or LL37), borneol, bismuth subgallate, antifungal pharmaceuticals, and antibiotics such as gentamycin, and streptomycin.

The top layer 101 and the adhesive skin contact layer 102 may be bonded to each other in those areas of both layers that extend beyond the contour of the absorbent pad 111. The top layer may also be bonded to the absorbent pad 111, e.g. by means of an adhesive, such as a pressure sensitive adhesive (e.g. a polyacrylate).

The dressing of the present disclosure is not limited to a specific shape, but any shape is conceivable, e.g. square, rectangular, oval, circular etc.

The maximum longitudinal extension, e.g. the length of the dressing may be from 3.0 to 50 cm, e.g. from 6.0 to 45 cm, e.g. from 7.0 to 40 cm.

The maximum lateral extension, e.g. the width of the dressing may be from 3.0 to 30 cm, e.g. from 4.0 cm to 20 cm.

In order to achieve sufficient adhesion properties, the border portion may have a width of from 10 to 50 mm, e.g. from 20 to 30 mm and extends along the contour of the absorbent pad 111.

A smaller sized dressing may have a smaller border portion than a larger sized dressing. This allows for easy handling and application of the product while still maintaining sufficient adhesion upon application.

The formation of protrusions in the release liner is not limited to a specific technique. The protrusions 106 may be formed by any suitable technique known to the skilled person.

In exemplary embodiments, and as illustrated in figures 1a-c, the protrusions 106 are embossed protrusions. Hence, the plurality of protrusions 106 are formed by embossing.

According to another aspect, there is provided a process for manufacturing a medical dressing 100 comprising:
a) providing a dressing 100 comprising a top layer 101 and an adhesive skin contact layer 102,
b) providing a release liner 103 having a first side 104 and an opposing second side 105, wherein at least a portion of the first side 104 of the release liner 103 comprises a plurality of protrusions 106 spaced apart from one another by non-protruding areas,
c) applying the release liner onto the adhesive skin contact layer such that the plurality of protrusions become detachably attached to the adhesive skin contact layer and unattached to the adhesive skin contact layer in the non-protruding areas.

The dressing may be provided by means known to the skilled person (step a). The dressing may also comprise an absorbent pad between the top layer and the adhesive skin contact layer. The assembly of the top layer, the adhesive layer, and the absorbent pad, where present, is not limited to a particular method, but any means (e.g. adhesive bonding, lamination etc.) may be utilized.

Preferably, the plurality of protrusions 106 on the first side 104 of the release liner 103 are formed as an in-line step in the process.

Accordingly, the process is significantly simplified, and is also advantageous from a storage perspective. This allows the release liner to be stored on e.g. a roll in a flat condition, requiring much less space than if the release liner would be provided with protrusions.

For example, the plurality of protrusions may be formed by embossing.

Embossing is a simple technique to form three dimensional features, e.g. protrusions in a layer. Furthermore, embossing is a suitable technique in embodiments where the protrusions are formed as an in-line step in the process.

For example, the release liner may be subject to an embossing tool on the second side of the release liner, which form protrusions 106 on the first side 104 and corresponding depressions 113 on the second side 105 of the release liner. The embossing tool may be arranged in the process line and embossed protrusions may be provided prior to application of the release liner to the adhesive skin contact layer 102.

The present disclosure is not limited to forming the protrusions by embossing.

In alternative embodiments, the protrusions may be formed by molding or casting.

In the various embodiments described hereinbefore, the top layer may be a film, sheet or membrane that is vapor permeable. Examples of suitable materials for the top layer include, but are not limited to polyurethane, polyethylene or polyamide films, silicone films, polyester based nonwoven materials, and laminates of polyester-based nonwoven materials and polyurethane films. Suitably, the top layer is a polyurethane film. The top layer may have a thickness of from 5 to 40 µm, e.g. from 15 to 25 µm. A thin layer of adhesive, such as a polyacrylate adhesive, may be applied to the top layer 101 to attach the top layer 101 to the adhesive skin contact layer 102 or, where present, an absorbent pad 111 or any other layer of the dressing.

The adhesive skin contact layer preferably comprises a silicone-based adhesive.

Examples of suitable silicone based adhesives include the two component RTV systems, such as Q72218 (Dow Corning), and SilGel 612 (Wacker Chemie AG) mentioned herein, as well as NuSil silicone elastomers. In embodiments of the invention the adhesive may comprise a soft silicone gel having a softness (penetration) of from 8 to 22 mm, e.g. from 12 to 17 mm, as measured by a method based on ASTM D 937 and DIN 51580, the method being described in European Patent Application No 14194054.4. The thickness of the silicone-based adhesive coating is typically at least 20 µm. The thickness of the silicone-based adhesive coating may be from 40 to 300 µm.

Preferably, the adhesive skin contact layer comprises a plurality of perforations.

If the adhesive skin contact layer comprises a polymeric film and a silicone based adhesive coating, the perforations extend through both of these layer.

The adhesive skin contact layer is arranged to receive body fluids, e.g. wound exudate from the wound while the absorbent pad functions to absorb the wound exudate and transport it away from the wound by evaporating it from the top of the dressing; i.e. through the top layer 101.

Terms, definitions and embodiments of all aspects of the present disclosure apply mutatis mutandis to the other aspects of the present disclosure.

Even though the present disclosure has been described with reference to specific exemplifying embodiments thereof, many different alterations, modifications and the like will become apparent for those skilled in the art.

Variations to the disclosed embodiments can be understood and effected by the skilled addressee in practicing the present disclosure, from a study of the drawings, the disclosure, and the appended claims. Furthermore, in the claims, the word "comprising" does not exclude other elements or steps, and the indefinite article "a" or "an" does not exclude a plurality.

### Example 1: Adhesion force between release liner and adhesive skin contact layer

Tests were performed to evaluate the adhesion force between the release liner and the adhesive skin contact layer of various samples. The adhesion force may also be referred to as the delamination force; i.e. the force required to remove the release liner from the adhesive skin contact layer.

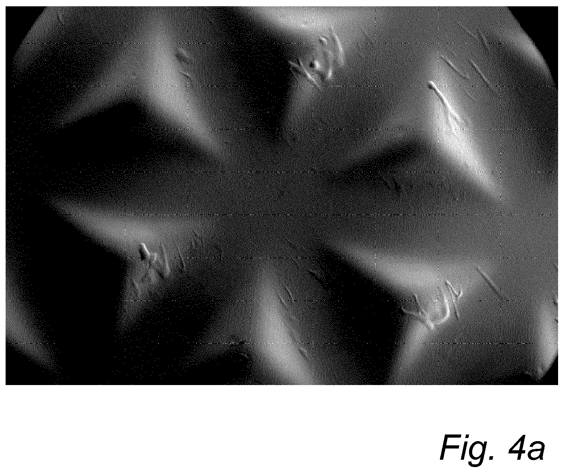

The dressing samples evaluated each contained a 20 µm polyurethane film as a the top layer, an absorbent pad having a thickness of about 3 mm, an adhesive skin contact layer comprising a polymeric film (a 20 µm polyurethane film) coated with a silicone based adhesive with varying thicknesses (see below), and a 100 µm polyethylene film as the release liner. The evaluated dressing samples were bordered dressing; i.e. the top layer and the adhesive skin contact layer were arranged to extend beyond the contour of the absorbent pad. The dressings tested differed by the thickness of the silicone adhesive coating, and the characteristics of the release liner, respectively.
Sample A: 50 gsm thick silicone adhesive coating, flat release liner
Sample B: 50 gsm thick silicone adhesive coating, release liner embossed with a pattern of pyramidal shaped protrusions (see figure 4A)
Sample C: 50 gsm thick silicone adhesive coating, release liner embossed with a pattern of protrusions as illustrated in figures 1b-1c and figure 2
Sample D: 200 gsm thick silicone adhesive coating, flat release liner
Sample E: 200 gsm thick silicone adhesive coating, release liner embossed with a pattern of pyramidal shaped protrusions (see figure 4A)
Sample F: 200 gsm thick silicone adhesive coating, release liner embossed with a pattern of protrusions as illustrated in figures 1b-1c and figure 2.

The silicone adhesive coating of samples A-F had the same constituents, but differed in tackiness (samples A-C had a higher tackiness than samples D-F).

The height of the protrusions for samples B and E was about 0.4 mm, whereas the height of the protrusions for samples C and F was about 1.0 mm. The protrusions of samples C and F had a substantially flat top surface (with a surface area of about 1.5 mm2 and an angle between the top surface and the sidewalls of about 90 degrees). The protrusions of samples B and E had a surface area of about 0.3 mm2 and an angle between the top surface and the sidewalls of about 45 degrees.

The samples were sent to sterilization on the production site to undergo typical sterilization cycle required for wound dressings (EtO sterilization).

The adhesion force was evaluated at the border portion of the dressing, and at the pad portion, respectively.

Each dressing sample was punched out with a punching tool to a size of 25x200 mm.

A vertical tensile tester with a flat grip, calibrated for speed 2500 mm/min and a head travel of 250 mm was utilized (crosshead speed: 2500 mm/min, gauge length: 50 mm).

The release liner was separated from the adhesive skin contact layer at the short side of each dressing sample, and the sample was attached with the release liner in the lower grip of the tensile tester.

The mean force was registered across the measuring length of 250 mm. The mean force used for the calculations was between 20 and 220 mm of the dressing sample; i.e. the first 20 mm and the last 30 mm of the sample were excluded. Accordingly, the mean force was calculated across a total length of 200 mm.

In each Sample category A-F, five dressing samples were tested.

The results are illustrated in table 1 below.

**Table 1: Adhesion force between release liner and adhesive skin contact layer**

| Samples | Average delamination force (N) - Border portion | Average delamination force (N) - Pad portion |
|---|---|---|
| Sample A | 2,47 | 1,63 |
| Sample B | 2,09 | 1,31 |
| Sample C | 0,72 | 0,45 |
| Sample D | 1,17 | 0,75 |
| Sample E | 1,36 | 0,67 |
| Sample F | 0,39 | 0,22 |

As illustrated in table 1, for samples A-C comprising a 50 gsm silicone adhesive coating, the delamination force was reduced when an embossed release liner was utilized. The delamination force was significantly reduced for Sample C. The delamination force of sample C was reduced at least 70% compared to the delamination force of sample A (both at the border portion and at the pad portion).

The same results were evident when comparing sample D with sample F having a thicker silicone adhesive coating.

The delamination force for sample E (comprising an embossed release liner) was actually larger than the delamination force for sample D (comprising a flat release liner). This may be explained by the thicker silicone adhesive layer having an enhanced tendency to follow the contour of the pyramidal shaped protrusions.

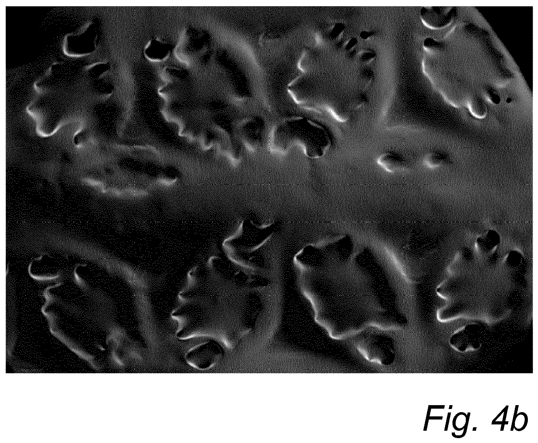

Figure 4b illustrates a Scanning electron microscopy (SEM) image of the skin-facing side (also referred to as the second side) of the adhesive skin contact layer of sample B. As illustrated in figure 4b, imprints from the sharper protrusions were formed.

For sample C, no imprints were formed in the adhesive skin contact layer.

Figures 4c and d illustrate SEM images of samples B, and C, respectively in cross-section.

As illustrated in figure 4d, the adhesive skin contact layer 102 lays flat on the top surface of the protrusions and does not sink into the gaps 108 defined between the release liner and the adhesive skin contact liner.

In contrast, the adhesive skin contact layer 102 of sample B conforms to the protrusions of the release liner.

Terms, definitions and embodiments of all aspects of the present disclosure apply mutatis mutandis to the other aspects of the present disclosure.

Even though the present disclosure has been described with reference to specific exemplifying embodiments thereof, many different alterations, modifications and the like will become apparent for those skilled in the art.

Variations to the disclosed embodiments can be understood and effected by the skilled addressee in practicing the present disclosure, from a study of the drawings, the disclosure, and the appended claims. Furthermore, in the claims, the word "comprising" does not exclude other elements or steps, and the indefinite article "a" or "an" does not exclude a plurality.

## Claims

1. A medical dressing (100) comprising a top layer (101), an adhesive skin contact layer (102) and a release liner (103) detachably attached to at least a portion of said adhesive skin contact layer (102); said release liner (103) having a first side (104) facing said adhesive skin contact layer (102) and an opposing second side (105), wherein said first side (104) of said release liner (103) comprises a plurality of protrusions (106) spaced apart from one another by non-protruding areas (107), wherein said protrusions (106) are detachably attached to said adhesive skin contact layer (102) and wherein said non-protruding areas (107) are unattached to said adhesive skin contact layer (102).

2. The medical dressing (100) according to claim 1, wherein said non-protruding areas (107) form gaps (108) between said release liner (103) and said adhesive skin contact layer (102).

3. The medical dressing (100) according to claim 1, wherein said first side of said release liner is detachably attached to from 10 to 80 %, preferably from 15 to 70%, most preferably from 20 to 50% of the surface area said adhesive skin contact layer (102).

4. The medical dressing (100) according to any one of the preceding claims, wherein each of the plurality of protrusions (106) has a substantially flat top surface (109); said top surface (109) forming the contact surface for detachable attachment of said release liner (103) to said adhesive skin contact layer (102).

5. The medical dressing (100) according to any one of the preceding claims, wherein each of said plurality of protrusions (106) has a top surface (109) and sidewalls (110) extending between said top surface (109) and said non-protruding area (107), wherein the angle, α, between said top surface (109) and said sidewalls (110) is from 60 to 120 degrees, preferably from 75 to 105 degrees, most preferably from 85 to 95 degrees.

6. The medical dressing (100) according to any one of the preceding claims, wherein the height of each of said protrusions (106) is from 0.3 to 2.0 mm, preferably from 0.5 to 1.5 mm.

7. The medical dressing (100) according to any one of the preceding claims, wherein the surface area of said top surface (109) of each of said protrusions (106) is from 0.8 to 3.0 mm2, preferably from 1.0 to 2.0 mm2.

8. The medical dressing (100) according to any one of the preceding claims, wherein said adhesive skin contact layer (102) comprises a silicone-based adhesive coating.

9. The medical dressing (100) according to any one of the preceding claims, wherein said adhesive skin contact layer (102) comprises a polymeric film provided with a silicone-based adhesive coating.

10. The medical dressing (100) according to claim 8 or claim 9, wherein said silicone-based adhesive coating has a thickness of from 35 to 300 gsm, e.g. from 45 to 250 gsm.

11. The medical dressing (100) according to any one of the preceding claims, wherein said dressing (100) further comprises an absorbent pad (111) arranged between said top layer (101) and said adhesive skin contact layer (102), wherein said top layer (101) and said adhesive skin contact layer (102) are configured to extend beyond the contour of said absorbent pad (111) to define a border portion along the contour of said absorbent pad (111).

12. The medical dressing (100) according to any one of the preceding claims, wherein said plurality of protrusions (106) are formed by embossing.

13. A process for manufacturing a medical dressing (100) comprising:
a) providing a dressing (100) comprising a top layer (101) and an adhesive skin contact layer (102),
b) providing a release liner (103) having a first side (104) and an opposing second side (105), wherein at least a portion of said first side (104) of said release liner (103) comprises a plurality of protrusions (106) spaced apart from one another by non-protruding areas (107);
c) applying said release liner (103) onto said adhesive skin contact layer (102) such that said plurality of protrusions (106) become detachably attached to said adhesive skin contact layer (102) and unattached to said adhesive skin contact layer (102) in said non-protruding areas (107).

14. The process according to claim 13, wherein said protrusions (106) on said first side (104) of said release liner (103) are formed as an in-line step in said process.

15. The process according to claim 13 or claim 14, wherein said plurality of protrusions on said first side (104) of said release liner (103) are formed by embossing.
